# FASCICULE DE BREVET EUROPEEN

(11) **EP 2 748 175 B1**
(45) Date de publication et mention de la délivrance du brevet: **07.10.2015**
(21) Numéro de dépôt: 12750394.4
(22) Date de dépôt: 24.08.2012
(51) Int. Cl.: C07H 1/00

(54) **PROCEDE D'ISOMERISATION DU GLUCOSE EN FRUCTOSE**
VERFAHREN ZUR ISOMERISIERUNG VON GLUKOSE ZU FRUKTOSE
METHOD FOR THE ISOMERISATION OF GLUCOSE INTO FRUCTOSE

(30) Priorité: 26.08.2011 FR 1157575
(43) Date de publication de la demande: 02.07.2014
(73) Titulaire: Centre National de la Recherche Scientifique (CNRS), 75016 Paris (FR); Université Claude Bernard de Lyon 1, 69100 Villeurbanne (FR)
(72) Inventeur: ESSAYEM, Nadine, F-38540 Saint Just Chaleyssin (FR); LOPES DE SOUZA, Rodrigo, CEP- Rio de Janeiro RJ (BR); RATABOUL, Franck, F-69008 Lyon (FR); FECHE, Cyril, F-01150 Leyment (FR); CARDOSO, Dilson, 13564-050 Sao Carlos-SP (BR); FABIANO, Demian, CEP 36420-000 Ouro Branco-MG (BR)
(74) Mandataire: Grosbois, Mathilde
(86) Numéro de dépôt international: PCT/EP2012/066546
(87) Numéro de publication internationale: WO 2013/030132

(56) Documents cités:
- WO-A1-2010/101024
- LIMA ET AL: "Isomerization of d-glucose to d-fructose over metallosilicate solid bases", APPLIED CATALYSIS A: GENERAL, ELSEVIER SCIENCE, AMSTERDAM, NL, vol. 339, no. 1, 17 janvier 2008 (2008-01-17), pages 21-27, XP022542128, ISSN: 0926-860X, DOI: 10.1016/J.APCATA.2007.12.030
- MOREAU C ET AL: "Isomerization of glucose into fructose in the presence of cation-exchanged zeolites and hydrotalcites", APPLIED CATALYSIS A: GENERAL, ELSEVIER SCIENCE, AMSTERDAM, NL, vol. 193, no. 1-2, 28 février 2000 (2000-02-28), pages 257-264, XP004272198, ISSN: 0926-860X, DOI: 10.1016/S0926-860X(99)00435-4
- ATSUSHI TAKAGAKI ET AL: "A one-pot reaction for biorefinery: combination of solid acid and base catalysts for direct production of 5-hydroxymethylfurfural from saccharides", CHEMICAL COMMUNICATIONS, no. 41, 1 janvier 2009 (2009-01-01), page 6276, XP055015326, ISSN: 1359-7345, DOI: 10.1039/b914087e

## Description

La présente invention concerne un procédé d'isomérisation du glucose en fructose. La présente invention concerne également un procédé de préparation de HMF (5-hydroxyméthylfurfural) à partir de glucose comprenant une étape d'isomérisation du glucose en fructose.

Le fructose possède de nombreuses applications, notamment dans le cadre de l'agro-alimentaire, le fructose présentant un pouvoir sucrant plus important que celui du glucose, et dans la préparation d'intermédiaires, par exemple le HMF, pour la préparation de polymères, notamment polyamides ou polyesters. Cependant, la production de fructose reste encore faible par rapport à la production du glucose.

Des procédés ont été développés pour permettre la préparation du fructose par isomérisation du glucose. Le procédé habituel est un procédé enzymatique, l'isomérisation du glucose en fructose étant catalysée par une enzyme immobilisée, la xylose isomérase. Cependant, il est nécessaire de mettre en oeuvre des quantités importantes d'enzyme et le coût lié à ce procédé est élevé. De plus, le temps de réaction pour obtenir une conversion et un rendement en fructose convenable est très important.

Afin d'améliorer ce procédé enzymatique il a été proposé (Yu et al., Process Biochemistry, 2011, 46, 599-603) de le mettre en oeuvre sous irradiation micro-onde. Cependant, le coût lié à ce type de procédé reste élevé par rapport aux résultats obtenus.

Des procédés ne mettant pas en oeuvre des micro-organismes ont également été développés. On connaît notamment des procédés mis en oeuvre en présence de soude en tant que catalyseur. Cependant, les résultats obtenus restent peu satisfaisants en raison notamment d'une perte rapide de sélectivité au cours du temps due aux réactions secondaires de type aldolisation. D'autre part, les bases homogènes génèrent des effluents aqueux à retraiter. Des procédés en catalyse hétérogène ont alors été proposés. On connaît notamment de FR2862973 l'utilisation d'aluminate de potassium pour catalyser la réaction d'isomérisation du glucose en fructose. Cependant, les résultats obtenus en termes de rendement, de conversion et de sélectivité restent faibles. On connaît également de Moreau et al (Applied Catalysis A: General, 193, 2000, 21-27) l'utilisation de zéolithe ou d'hydrotalcite (catalyseur à base d'aluminium et de magnésium) comme catalyseur de la réaction d'isomérisation. Cependant, les meilleures sélectivités en fructose (90%) sont obtenues pour de faibles conversions de glucose (10 à 20%). On connaît également de Valente et al. (Applied Catalysis A : General, 339, 2008, 21-27) l'utilisation de métallosilicates comme catalyseur de la réaction d'isomérisation. Des procédés ont également été développés en catalyse acide (Davis et al., Angew. Chem. Int. Ed., 2010, 49, 8954-8957) qui utilisent des catalyseurs Sn-Beta. Les résultats obtenus par ces procédés ne sont cependant pas suffisamment bons pour concurrencer le procédé enzymatique actuel.

Enfin, il est connu de Ebitani et al (Chem. Commun., 2009, 6276-6278) la préparation de HMF à partir du glucose en une seule étape comprenant l'isomérisation du glucose en fructose en présence d'un catalyseur basique solide et la déshydratation du fructose en HMF en présence d'un catalyseur acide solide.

Cependant, ces différents procédés présentent l'inconvénient d'être trop couteux ou de ne pas présenter de bons résultats en termes de conversion du glucose, rendement en fructose et sélectivité en fructose.

L'objectif de la présente invention est de fournir un procédé d'isomérisation du glucose en fructose qui soit industrialisable et qui puisse être mis en oeuvre à un coût réduit.
Un autre objectif de la présente invention est de fournir un procédé présentant de bons résultats en termes de conversion du glucose, de rendement en fructose et de sélectivité en fructose.
Un autre objectif encore de la présente invention est de fournir un procédé d'isomérisation du glucose en fructose permettant d'obtenir de bons résultats en termes de rendement, conversion et sélectivité en un temps réduit.
Un autre objectif de l'invention est de fournir un tel procédé qui soit plus robuste que les procédés enzymatiques, qui sont sensibles à de nombreux facteurs tels que le pH, la température, la présence d'inhibiteurs, et qui puisse donc être plus facilement mis en oeuvre même sur des matières premières de pureté variable issues de la biomasse.
Un autre objectif de la présente invention est également de fournir un procédé de préparation de l'HMF à partir de glucose.
D'autres objectifs encore apparaîtront à la lecture de la description qui suit.

Tous les inconvénients de l'art antérieur sont résolus par la présente invention qui concerne un procédé d'isomérisation du glucose en fructose dans l'eau en présence d'un catalyseur basique solide, préférentiellement utilisé sans activation thermique de décarbonatation, caractérisé par la réversibilité des isothermes d'adsorption du CO₂ à basse température, notamment à 30°C et de préférence une chaleur différentielle d'adsorption du CO₂, mesurée à 30°C, comprise entre 60 et 110 kJ.mol⁻¹, de façon préférée entre 75 et 90kJ.mol⁻¹, le catalyseur étant un catalyseur comprenant au moins un oxyde de Lanthanide supporté ou non supporté ou un tamis moléculaire à base de silicium contenant son template organique.

De préférence, le catalyseur est choisi parmi les catalyseurs bases faibles solides. Ces bases faibles sont caractérisées par des chaleurs différentielles d'adsorption du CO₂ mesurée à 30°C entre 60 et 110 kJ. mol⁻¹, e façon préférée entre 75 et 90kJ.mol⁻¹. Ces catalyseurs sont utilisables sans activation thermique de décarbonatation, ce qui est l'inconvénient majeur qui limite l'application des catalyseurs basiques usuels au niveau industriel.

La réversibilité de l'adsorption du CO₂ se traduit notamment par une résistance du catalyseur à l'empoisonnement par le CO₂. Cette propriété n'est pas conditionnée à une activation préalable. Il faut savoir en effet que, de manière générale, les catalyseurs tels que ceux utilisés dans l'art antérieur, qui comportent des sites basiques, lorsqu'ils sont laissés à l'air ambiant, sont empoisonnés par le CO₂, celui-ci se fixant sur les sites basiques du catalyseur pour former des espèces carbonatées. On entend par sites basiques une espèce contenant une paire d'électrons qui peut être transférée à une molécule acide. Il est donc usuellement nécessaire de prétraiter ces catalyseurs, par exemple à haute température, pour les activer et de les utiliser dans un milieu réactionnel décarbonaté ou suite à l'activation thermique, de les réhydrater de façon à limiter l'inhibition des sites basiques du solide par des traces de CO₂ atmosphérique. Au contraire, les catalyseurs de l'invention ne nécessitent pas de prétraitement, notamment de prétraitement à haute température.

La réversibilité de l'adsorption de CO₂ par un catalyseur peut être déterminée par calorimétrie couplée aux mesures d'isothermes d'adsorption du CO₂ (totale et réversible) par mesure volumétrique. Les mesures d'adsorption du CO₂ sont réalisées au moyen d'un calorimètre, notamment d'un calorimètre Tian Calvet, et elles sont couplées aux mesures d'isothermes d'adsorption du CO₂ au moyen d'un équipement volumétrique en verre équipé d'une jauge de pression de précision. Le solide, ntroduit dans la cellule en verre, est placée dans le calorimètre maintenue à la température de mesure (30 °C) après avoir été prétraité sous vide à cette température jusqu'à l'obtention d'un bon vide (10⁻⁵ torrs) ou à une température supérieure. Le CO₂ est introduit par petits incréments à 30°C, la chaleur dégagée est mesurée à chaque dose de CO₂ tout comme la pression d'équilibre. L'isotherme d'adsorption est déterminée jusqu'à la pression d'équilibre de 1 Torr. Après la mesure de la première isotherme (adsorption totale), une seconde isotherme est réalisée (adsorption réversible) après avoir traité sous vide le catalyseur solide à 30°C pendant 15h.

Les catalyseurs comprenant un oxyde de Lanthanide supporté ou non supporté ou un tamis moléculaire à base de silicium contenant leur template (notamment CTA : cetyltrimethylammonium bromide ou bromure de cétyltriméthylammonium) utilisés dans l'invention sont donc ceux qui présentent cette réversibilité de l'adsorption de CO₂ à 30 °C associée à une chaleur différentielle d'adsorption comprise entre 60 et 110 kJ.mol⁻¹, de façon préférée entre 75 et 90kJ.mol⁻¹.

Ainsi, et de manière avantageuse, le procédé de l'invention ne comprend pas de prétraitement du catalyseur avant utilisation, destiné à réactiver le catalyseur, notamment de prétraitement à haute température.

De préférence, la caractéristique de résistance du catalyseur à l'empoisonnement par le CO₂ se traduit par le fait que la force basique du catalyseur se trouve entre la chimisorption et la physisorption. De préférence au moins 50% des sites basiques du catalyseur, en dehors de tout prétraitement, notamment en dehors de tout prétraitement à haute température, présentent une chaleur d'adsorption du CO₂ comprise entre 60 et 110, de préférence entre 75 et 90 kJ.mol⁻¹. Par « chaleur différentielle d'adsorption du CO₂ », on désigne la chaleur molaire dégagée par l'adsorption de doses infinitésimales de CO₂, à température constante, sur le catalyseur initialement sous vide dans un calorimètre de type Tian Calvet. Les valeurs de chaleurs différentielles d'adsorption de CO₂ correspondent à la valeur du plateau de la courbe représentant la variation des chaleurs différentielles (Q diff kJ.mol⁻¹) en fonction de la quantité de CO₂ adsorbé si le solide basique présente des sites homogènes en force basique. Si les chaleurs différentielles diminuent avec le recouvrement en CO₂, la valeur considérée est la moyenne des chaleurs différentielles d'adsorption à 50% de recouvrement en CO₂.

Le catalyseur peut être choisi parmi les oxydes de Lanthanides, supportés ou non, éventuellement hydroxylés et/ou carbonatés, les oxydes mixtes de Lanthanides avec d'autres métaux, supportés ou non, éventuellement hydroxylés et/ou carbonatés, ou les tamis moléculaires à base de silicium contenant leur template organique.

Dans un mode de réalisation, le catalyseur est constitué d'un oxyde de Lanthanide, de préférence de La, supporté ou non supporté.

Les Lanthanides peuvent être oxydés à des degrés divers ; ils peuvent être partiellement hydroxylés et/ou carbonatés. On peut citer notamment : La₂O₃, La(OH)₃ sur charbon, La₂O_{3-x-y}(CO₃)ₓ(OH)_{2y}, formule dans laquelle x peut être 0, 1, 2 ou 3, par exemple 0,1 ou 2, y peut être 0, 1, 2 ou 3 avec 0≤x+y≤3.

Dans un mode de réalisation, le catalyseur comprend un oxyde de Lanthanide, de préférence de La, supporté ou non. Dans un mode de réalisation préféré, ce catalyseur, supporté ou non supporté, est un oxyde mixte de Lanthanide, de préférence de La, avec d'autres métaux, notamment des métaux alcalins ou alcalino-terreux, e.g. Mg. On peut citer l'oxyde mixte de La et de Mg, e.g. MgLaO. Ces oxydes mixtes peuvent comprendre un oxyde de Lanthanide partiellement hydroxylé et/ou carbonaté, par exemple de formule ci-dessus La₂O_{3-x-y}(CO₃)ₓ(OH)_{2y}, et par exemple avec un oxyde de Mg (e.g. MgO).

On entend par Lanthanides ou terres rares (Ln), les éléments chimiques choisis dans le groupe constitué par le scandium, l'yttrium, et les éléments chimiques de numéro atomique 57 à 71, de préférence, les éléments chimiques choisis dans le groupe constitué par le scandium et les éléments chimiques de numéro atomique 57 à 71, de préférence les éléments chimiques de numéro atomique 57 à 71. De façon avantageuse les Lanthanides sont choisis parmi le cérium (Ce), le lanthane (La), le praséodyme (Pr), le néodyme (Nd), l'yttrium (Y) et le gadolinium (Gd), le samarium (Sm) et l'holmium (Ho), de préférence le cérium (Ce), le lanthane (La), le praséodyme (Pr), le néodyme (Nd), et le gadolinium (Gd), le samarium (Sm) et l'holmium (Ho). On choisit de préférence le lanthane.

Les supports peuvent notamment être un support carboné (charbon actif, graphite, charbon ou graphite fonctionnalisé), les oxydes métalliques, par exemple les oxydes de titane ou l'alumine. De préférence, les supports sont des supports stables en condition hydrothermales.

Des catalyseurs comprenant au moins un oxyde de Lanthanide préférés sont :
- MgLaO (oxyde mixte à base de magnésium et de lanthane), notamment tel que décrit dans J Catalysis 2002 211, 150 ou dans Catalysis Today 152 (2010)110-114 comprenant notamment du MgO et de l'hydroxy-carbonate de La ;
- LaO (oxyde simple de lanthane), supporté ou non, notamment supporté, de préférence sur support carboné.

Du fait de leurs procédés de fabrication, les tamis moléculaires incluent généralement des molécules organiques qui ont servi à leur synthèse. Ces molécules organiques sont dénommées « templates organiques » et les tamis moléculaires selon l'invention peuvent comporter les templates organiques. Le template organique (notamment CTA : cetyltrimethylammonium bromide) est indispensable au développement de la basicité des tamis : le site basique est l'oxygène du réseau situé en position de contre-anion de l'ammonium à l'entrée des pores.

De préférence, les catalyseurs de type tamis moléculaire sont des tamis moléculaires à base de silicium, notamment ceux de la famille M41 S. Les catalyseurs de type tamis moléculaire préférés sont ceux du type MCM48 et MCM50 utilisés en présence de leur template organique CTA, tels ceux décrits dans Micropor. Mesopor. Mater.70(2004)135.

De préférence, dans le procédé de la présente invention, la quantité de catalyseur est comprise entre 0,5 et 100% en poids, de préférence entre 1 et 50% en poids, notamment entre 2 et 25% en poids, par exemple entre 5 et 10% en poids, par rapport au poids de glucose.

De préférence, la quantité de glucose est comprise entre 0,5 et 15% en poids, de préférence entre 1 et 10% en poids, par rapport au poids d'eau.

Le procédé de la présente invention peut être mis en oeuvre à une température comprise entre 75 et 180 °C, de préférence entre 80 et 150 °C, par exemple à entre 85 °C et 100 °C, notamment à 100 °C environ. De manière avantageuse, le procédé peut être donc mis en oeuvre à des températures peu élevées, notamment comprises entre 85 °C et 100 °C. Ceci est notamment rendu possible par le fait que le catalyseur est résistant à l'empoisonnement par le CO₂ et présente donc une réversibilité de l'adsorption du CO₂ à faible température. Au contraire, pour les catalyseurs ne résistant pas à l'empoisonnement par le CO₂, le rendement de l'isomérisation sera très faible voire nulle à de telles températures.
Le catalyseur est mis en oeuvre en absence d'activation thermique en particulier.
Le procédé de l'invention peut également être mis en oeuvre à pression atmosphérique ou sous pression d'un gaz inerte, par exemple hélium, jusqu'à une pression de 2 MPa environ (20 bars).

De façon avantageuse, le catalyseur peut être facilement récupéré en fin de réaction par toute méthode connue de l'homme du métier, notamment par simple filtration. Il est ainsi possible de récupérer le catalyseur et de le réutiliser pour une nouvelle isomérisation du glucose en fructose. De manière particulièrement avantageuse, le catalyseur peut être réutilisé tel quel, sans prétraitement particulier, dans 2, 3, 4, voire plus, réactions d'isomérisation du glucose en fructose.

Dans un mode de réalisation particulier, la réaction peut également être mise en oeuvre en présence d'eau et d'un solvant, notamment un solvant fortement polaire aprotique, par exemple le DMSO (diméthylsulfoxyde) de DMF (diméthylformamide) NMP (N-méthylpyrrolidone).
Le catalyseur basique solide peut éventuellement subir un traitement avant réaction par de la pyridine, par une base forte en général comme par exemple la lutidine, l'ammoniac, etc. Sans vouloir être lié par une quelconque théorie ce traitement permettrait de bloquer les éventuels sites acides de Lewis du catalyseur. Ce traitement s'apparente donc à un empoisonnement du catalyseur. Ce traitement permet d'améliorer les résultats en termes de conversion du glucose, rendement en fructose et sélectivité en fructose. Ce traitement à la pyridine permet notamment d'améliorer la sélectivité en fructose.

De façon avantageuse, le procédé de l'invention permet d'obtenir des conversions en glucose de l'ordre de entre 20 et 50%, de préférence de l'ordre de 40, des rendements en fructose de l'ordre de 20 et 35 %, de préférence de l'ordre de 32, et des sélectivités en fructose de l'ordre de 70 et 90%, de préférence de l'ordre de 80 %.

De façon avantageuse, le procédé de l'invention permet d'obtenir de tels résultats en un temps très court. Notamment, et de façon surprenante, le procédé de l'invention peut être conduit à son terme, notamment permet d'obtenir de tels résultats de conversion, rendement et sélectivité, en moins de 5 heures, de préférence en moins de 2 heures, notamment en 1 heure environ, alors que les procédé enzymatiques actuels nécessitent plus de 24 heures.
Le système catalytique présente l'avantage par rapport aux autres bases solides usuelles d'être mis en oeuvre en absence de prétraitement, thermique notamment, ce qui est avantageux pour une application industrielle.

L'invention concerne également un procédé de préparation de HMF à partir du glucose comprenant les étapes suivantes :
i) isomérisation du glucose en fructose selon le procédé de l'invention ;
ii) ajout d'acide carboxylique au milieu réactionnel obtenu à l'étape i) ;
iii) récupération du HMF.

Les acides carboxyliques de l'étape (ii) peuvent être des monoacides, des diacides ou des triacides. Ils sont notamment choisis parmi :
- les acides de formule R-COOH dans laquelle R représente un atome d'hydrogène ou une chaîne alkyle, linéaire ou ramifiée, en C₁ à C₅, de préférence en C₁ à C₃, éventuellement substituée par un ou plusieurs groupes OH ;
- les acides de formule HOOC-L-COOH dans laquelle L représente une liaison ou une chaîne alkyle, linéaire ou ramifiée, en C₁ à C₅, de préférence en C₁ à C₃, éventuellement substituée par un ou plusieurs groupes OH et/ou COOH ; ou
- leurs mélanges.

De préférence, l'acide carboxylique de l'étape (ii) est un monoacide choisi parmi les acides de formule R-COOH dans laquelle R représente un atome d'hydrogène ou une chaîne alkyle, linéaire ou ramifiée, en C₁ à C₅, de préférence en C₁ à C₃, éventuellement substituée par un ou plusieurs groupes OH.

De préférence, l'acide carboxylique est l'acide formique, l'acide acétique, l'acide malique, l'acide citrique, l'acide oxalique, l'acide lactique ou leurs mélanges. De préférence, l'acide carboxylique est l'acide formique, l'acide acétique, l'acide lactique ou leurs mélanges. De façon plus préférée, l'acide carboxylique est l'acide formique, l'acide acétique ou leur mélange. De façon encore plus particulièrement préférée, l'acide carboxylique est l'acide acétique.

L'homme du métier, selon qu'il préfère favoriser la conversion du fructose ou la sélectivité en HMF ou avoir un bon compromis entre ces deux caractéristiques pourra déterminer la proportion et la nature de l'acide à intégrer au milieu réactionnel. La quantité d'acide ne doit pas être trop importante au risque d'augmenter la production de produits secondaires, notamment d'humine. Ainsi, de préférence la quantité d'acide est inférieure à 80 % en poids, généralement comprise entre 5 et 80 % en poids, notamment comprise entre 10 et 60 % en poids, par exemple environ 50%, 20 % ou 10 % en poids par rapport au poids du milieu réactionnel liquide.

L'étape (ii) peut être menée à une température comprise entre 100 et 200 °C, de préférence entre 120 et 180 °C, par exemple entre 150 et 180 °C.

L'étape (ii) peut également être mise en oeuvre à pression atmosphérique ou sous pression d'un gaz inerte, par exemple l'hélium, jusqu'à une pression de 3,5 MPa environ (soit 35 bars).

L'étape (ii) peut avantageusement être mise en oeuvre en présence d'un catalyseur acide hétérogène. De préférence, le catalyseur est choisi parmi l'acide 12-tungstophosphorique, de préférence dispersé sur hydroxyde de niobium (NbOH); l'hydroxyde de niobium ; les zeolithes telles H-ZSM-5 ou H-Y, des argiles cationiques de type K10, les charbons sulfonés ; les charbons fonctionnalisés, par exemple par des groupements carboxyliques, par exemple suite à une oxydation, par exemple oxydation par de l'eau de javel ; ou leurs mélanges. De préférence, le catalyseur est choisi parmi l'acide 12-tungstophosphorique, de préférence dispersé sur hydroxyde de niobium (NbOH); l'hydroxyde de niobium ; les zeolithes telles H-ZSM-5 ou H-Y, des argiles cationiques de type K10,les charbons sulfonés ; les charbons fonctionnalisés, par exemple par des groupements carboxyliques, par exemple suite à une oxydation, par exemple oxydation par de l'eau de javel ; ou leurs mélanges. De préférence, le catalyseur est choisi parmi l'acide 12-tungstophosphorique, de préférence dispersé sur hydroxyde de niobium (NbOH); l'hydroxyde de niobium ; les zircones sulfatées; les sels acides de césium de l'acide 12-tungstophosphorique (Cs₂HPW₁₂O₄₀); le dioxyde de titane ; les charbons sulfonés ; les charbons fonctionnalisés, par exemple par des groupements carboxyliques, par exemple suite à une oxydation, par exemple oxydation par de l'eau de javel ; ou leurs mélanges. L'ajout de ces catalyseurs permet notamment de manière avantageuse d'augmenter la conversion en fructose, et la sélectivité en HMF. De façon particulièrement préférée, le catalyseur est un charbon sulfoné ou un charbon fonctionnalisé.

Lorsqu'il est présent, la quantité de catalyseur est de préférence comprise entre 2 et 100% en poids, de préférence entre 2 et 10 % en poids, par exemple 5 % en poids par rapport au poids de glucose de départ.

La présente invention va maintenant être décrite à l'aide d'exemples non limitatifs pris en référence au dessin sur lequel :
La figure 1 représente les courbes calorimétriques d'adsorption du CO₂ pour un catalyseur ZrCs activé sous vide à 450 °C pendant 2 heures, lors de la première adsorption dite totale (carrés noirs), puis lors de la deuxième adsorption, dite réversible (carrés blancs).
La figure 2 montre les isothermes d'adsorption du CO₂ mesurées parallèlement à la mesure des chaleurs différentielles d'adsorption du CO₂ avec le recouvrement en CO₂ lors de la première et de la deuxième adsorption du CO₂ (respectivement adsorptions totale et réversible).
La figure 3 représente les courbes calorimétriques d'adsorption du CO₂ pour un catalyseur ZrCs prétraité sous vide à 30°C pendant 24 heures, lors de la première adsorption dite totale (carrés noirs), puis lors de la deuxième adsorption, dite réversible (carrés blancs).
La figure 4 montre les isothermes d'adsorption du CO₂ mesurées parallèlement à la mesure des chaleurs différentielles d'adsorption du CO₂ avec le recouvrement en CO₂ lors de la première et de la deuxième adsorption du CO₂ (respectivement adsorptions totale et réversible).
La figure 5 représente les courbes calorimétriques d'adsorption du CO₂ (première adsorption totale) pour des catalyseurs [CTA]Si-MCM48, et [CTA]Si-MCM50.
La figure 6 représente les courbes calorimétriques d'adsorption du CO₂ pour un catalyseur [CTA]Si-MCM50 prétraité sous vide à 30°C pendant 24 heures, lors de la première adsorption dite totale (carrés noirs), puis lors de la deuxième adsorption, dite réversible (carrés blancs).
La figure 7 montre les isothermes d'adsorption du CO₂ mesurées parallèlement à la mesure des chaleurs différentielles d'adsorption du CO₂ avec le recouvrement en CO₂ lors de la première et de la deuxième adsorption du CO₂ (respectivement adsorptions totale et réversible).

### Exemple 1 : Isomérisation du glucose en fructose en présence de divers catalyseurs basiques solides à 100°C

La réaction d'isomérisation du glucose en fructose est réalisée dans un autoclave de 100ml en mettant en oeuvre une solution de 1% en poids de glucose dans l'eau. Les quantités suivantes sont introduites dans le réacteur : 30g d'eau distillée, 0,3g de glucose (1% en poids /eau), 0,015g de catalyseur (5% en poids / glucose). Aucune activation n'a été appliquée aux catalyseurs solides. 20 bars d'hélium sont introduits dans l'autoclave. Le milieu réactionnel est agité au moyen d'un agitateur magnétique. Le milieu réactionnel est porté à la température de réaction au moyen de résistances électriques régulées à 100°C. Après 2h de réaction à 100°C, l'autoclave est refroidi au moyen d'un bain de glace. La conversion du glucose et le rendement molaire en fructose sont déterminés par analyse HPLC-RID (colonne : COREGEL 87C).

L'isomérisation a été réalisée en présence de différents catalyseurs, à savoir :
a) catalyseurs selon l'invention :
   - MgLaO ;
   - MgLaO traité par la pyridine ;
   - MCM48 ;
   - MCM50.
b) catalyseurs de l'art antérieur :
   - Sn Beta ;
   - ZrCs ;
   - NaOH (dans ce cas particulier la réaction a été menée à 50°C).

L'isomérisation a également été réalisée selon le protocole ci-dessus en l'absence de catalyseur.

| Catalyseur | Conversion (%) | Rdt Fructose (%) | Rdt Mannose (%) | Rdt Autres (%) |
|---|---|---|---|---|
| MgLaO | 52,6 | 28,2 | 5,1 | 6,5 |
| MgLaO traité Pyridine | 28,2 | 22,1 | 2,1 | 2,6 |
| MCM48 | 22 | 17,5 | 2 | 2 |
| MCM50 | 16,4 | 13,6 | 0,8 | 0,4 |
| SnBeta | 2 | 1,8 | 0,2 | |
| ZrCs | 3,5 | 2,5 | 1 | 0 |
| *enzyme, (comparatif*)* | | 43 | | |
| NaOH,50°C | 45 | 32 | 4 | 6 |
| sans catalyseur | 0,6 | 0,4 | 0 | 0 |

| | | | | |
|---|---|---|---|---|
| Rdt = rendement molaire *résultats reportés dans Dahai Yu et al. Process Biochemistry 46 (2011) 599-603 | | | | |

Les résultats montrent que les catalyseurs selon l'invention présentent une activité améliorée (notamment en termes de conversion en glucose, rendement en fructose et sélectivité en fructose) par rapport aux catalyseurs solides basiques de l'état de la technique, ne présentant pas de réversibilité d'adsorption de CO₂ comme par exemple ZrCs ou par rapport à un acide de Lewis Sn-Beta. Les résultats montrent également que la mise en oeuvre du procédé selon l'invention permet d'obtenir de meilleurs résultats ou des résultats similaires par rapport au procédé enzymatique ou aux procédés catalysés par des bases homogènes.
Enfin, les résultats montrent que le traitement du catalyseur avec de la pyridine permet d'améliorer la sélectivité en fructose.

### Exemple 2 : Isomérisation du glucose en fructose en présence de divers catalyseurs basiques solides à 100°C

La réaction d'isomérisation du glucose en fructose est réalisée dans un autoclave de 100ml en mettant en oeuvre une solution de 2% en poids de glucose dans l'eau. Les quantités suivantes sont introduites dans le réacteur : 30g d'eau distillée, 0,6g de glucose (2% en poids /eau), 0,030g de catalyseur (5% en poids / glucose). Aucune activation n'a été appliquée aux catalyseurs solides. 20 bars d'hélium sont introduits dans l'autoclave. Le milieu réactionnel est agité au moyen d'un agitateur magnétique. Le milieu réactionnel est porté à la température de réaction au moyen de résistances électriques régulées à 100 °C. Après 2h ou 1h** de réaction à 100 °C, l'autoclave est refroidi au moyen d'un bain de glace. La conversion du glucose et le rendement molaire en fructose sont déterminés par analyse HPLC-RID (colonne : COREGEL 87C).

L'isomérisation a été réalisée en présence de différents catalyseurs, à savoir :
- MgLaO (oxyde mixte à base de magnésium et de lanthane) (pendant 1 h et pendant 2h);
- LaO (oxyde simple de lanthane)/charbon (LaOH/C) ;
- ZrCs (comparatif) ;
- hydrotalcite (Mg/AI=3) pendant 1 h. (comparatif)

| Catalyseur | Conversion (%) | Rdt Fructose (%) | Rdt Mannose (%) | Rdt Autres (%) |
|---|---|---|---|---|
| 5%MgLaO | 35,61 | 25,57 | 4,05 | 3,32 |
| 5%LaOH/C | 51,72 | 25,97 | 6,95 | 5,68 |
| 5%ZrCs | 5,14 | 4,96 | 0,39 | 0 |
| 5%MgLaO** | 34,54 | 27,36 | 3,54 | 4,14 |
| 5%HDT Mg/Al=3** | 24,64 | 15,54 | 1,09 | 0 |

| | | | | |
|---|---|---|---|---|
| Rdt = rendement molaire, ** = temps de réaction 1 heure | | | | |

Les résultats montrent que les catalyseurs selon l'invention présentent une activité améliorée (notamment en termes de conversion en glucose, rendement en fructose et sélectivité en fructose) par rapport aux catalyseurs solides de l'état de la technique, ne présentant pas de réversibilité d'adsorption de CO2 (par exemple ZrCs). L'isomérisation mise en oeuvre selon le procédé de l'invention permet d'obtenir des résultats en termes de rendement, conversion en fructose et sélectivité en fructose amélioré par rapport au procédé mis en oeuvre en présence d'hydrotalcite Mg/AI.

### Exemple 3 : Isomérisation du glucose en fructose en présence de divers catalyseurs basiques solides à 150 °C

La réaction d'isomérisation du glucose en fructose est réalisée dans un autoclave de 100ml en mettant en oeuvre une solution de 1% en poids de glucose dans l'eau. Les quantités suivantes sont introduites dans le réacteur : 30g d'eau distillée, 0,3g de glucose (1% en poids /eau), 0,015g de catalyseur (5% en poids / glucose). Aucune activation n'a été appliquée aux catalyseurs solides. 20 bars d'hélium sont introduits dans l'autoclave. Le milieu réactionnel est agité au moyen d'un agitateur magnétique. Le milieu réactionnel est porté à la température de réaction au moyen de résistances électriques régulées à 150 °C. Après 2h de réaction à 150 °C, l'autoclave est refroidi au moyen d'un bain de glace. La conversion du glucose et le rendement molaire en fructose sont déterminés par analyse HPLC-RID (colonne : COREGEL 87C).

L'isomérisation a été réalisée en présence de différents catalyseurs, à savoir :
- MgLaO ;
- Sn-Beta (comparatif) ;
- ZrCs traité sous vide (sv) à 450 °C pendant 2h, ce traitement étant habituellement réalisé pour activer le catalyseur (comparatif) ;
- ZrCs (comparatif).

L'isomérisation a également été réalisée selon le protocole ci-dessus en l'absence de catalyseur.

| Catalyseur | Conversion (%) | Rdt Fructose (%) | Rdt Mannose (%) | Rdt Autres (%) |
|---|---|---|---|---|
| 5%MgLaO | 59,4 | 23,0 | 6,9 | 5,1 |
| 5%Sn-Beta | 15,8 | 9,9 | 1,4 | 1,2 |
| 5%ZrCs traite sv à 400 °C/2h | 14,4 | 8,4 | 1,6 | 1,2 |
| sans cata | 7,2 | 3,5 | 0,5 | 2,3 |
| 5%ZrCs | 13,3 | 5,8 | 2,9 | 2,4 |

| | | | | |
|---|---|---|---|---|
| Rdt = rendement molaire | | | | |

Les résultats montrent que le catalyseur ZrCs, caractérisé par une adsorption de CO₂ irréversible à 30 °C et par la présence de sites basiques forts en particulier après une activation sous vide à 450 °C est plus actif à 150 °C par rapport à 100 °C mais reste sensiblement moins actif que MgLaO, catalyseur conforme à l'invention. Les résultats montrent que le prétraitement de ZrCs ne permet pas d'améliorer l'activité du catalyseur. Les résultats montrent aussi que le catalyseur Sn-Beta, qui est un acide de Lewis est plus actif à 150 °C par rapport à 100 °C mais reste sensiblement moins actifs que MgLaO, catalyseur conforme à l'invention.

### Exemple 4 : Isomérisation du glucose en fructose comparaison MgLaO avec aluminate à 100°C

La réaction d'isomérisation du glucose en fructose est réalisée dans un autoclave de 100ml en mettant en oeuvre une solution de 2% en poids de glucose dans l'eau. Les quantités suivantes sont introduites dans le réacteur : 30g d'eau distillée, 0,6g de glucose (2% en poids /eau), 0,060g de catalyseur (10% en poids / glucose). Aucune activation n'a été appliquée aux catalyseurs solides. 20 bars d'hélium sont introduits dans l'autoclave. Le milieu réactionnel est agité au moyen d'un agitateur magnétique. Le milieu réactionnel est porté à la température de réaction au moyen de résistances électriques régulées à 100 °C. Après 1h de réaction à 100 °C, l'autoclave est refroidi au moyen d'un bain de glace. La conversion du glucose et le rendement molaire en fructose sont déterminés par analyse HPLC-RID (colonne: COREGEL 87C).

L'isomérisation a été réalisée en présence de 10% de MgLaO en poids par rapport au poids de glucose et de 10% d'aluminate de sodium (comparatif) en poids par rapport au poids de glucose.

| catalyseur | Conversion (%) | Rdt Fructose (%) | Rdt Autres (%) |
|---|---|---|---|
| 10%MgLaO | 38,7 | 31,2 | 8,6 |
| 10%aluminate de sodium | 61,4 | 28,4 | 17,0 |

| | | | |
|---|---|---|---|
| Rdt = rendement molaire | | | |

Les résultats montrent que MgLaO dans le procédé de l'invention permet de limiter la formation de sous-produits. En effet, bien que la conversion du glucose soit plus faible dans le procédé de l'invention que dans le procédé catalysé par des aluminates de sodium, le rendement en fructose et la sélectivité en fructose y sont plus élevés.

### Exemple 5 : Isomérisation du glucose en fructose catalysé par MgLaO influence de la température

La réaction d'isomérisation du glucose en fructose est réalisée dans un autoclave de 100ml en mettant en oeuvre une solution de 1% en poids de glucose dans l'eau. Les quantités suivantes sont introduites dans le réacteur : 30g d'eau distillée, 0,3g de glucose (1% en poids /eau), 0,015g de catalyseur (5% en poids / glucose). Aucune activation n'a été appliquée au catalyseur solide. 20 bars d'hélium sont introduits dans l'autoclave. Le milieu réactionnel est agité au moyen d'un agitateur magnétique. Le milieu réactionnel est porté à la température de réaction au moyen de résistances électriques régulées à la température de réaction. Après 2h de réaction à la température de réaction, l'autoclave est refroidi au moyen d'un bain de glace. La conversion du glucose et le rendement molaire en fructose sont déterminés par analyse HPLC-RID (colonne : COREGEL 87C). L'isomérisation a été réalisée en présence de 5% de MgLaO en poids par rapport au poids de glucose à 85 °C (pendant 13 heures), 100 °C, 150 °C et 180 °C.

| Température | Conversion (%) | Rdt Fructose (%) | Rdt Autres (%) |
|---|---|---|---|
| 85 °C** | 37,5 | 26,6 | 6,8 |
| 100 °C | 52,6 | 28,2 | 11,6 |
| 150 °C | 59,4 | 23,0 | 12,1 |
| 180 °C | 78.0 | 8,1 | 28,2 |

| | | | |
|---|---|---|---|
| Rdt = rendement molaire, ** 13 heures | | | |

Les résultats montrent que plus la température augmente plus la conversion du glucose est importante. Cependant, cette augmentation de la température se traduit également par une augmentation de l'apparition de sous-produits et par conséquent une diminution du rendement en fructose et donc de la sélectivité en fructose.
Ainsi, le meilleur compromis en termes de conversion du glucose, rendement et sélectivité en fructose sont obtenus pour des températures inférieures à 150 °C et notamment à 100 °C.

### Exemple 6 : Isomérisation du glucose en fructose catalysé par MgLaO influence de la concentration en glucose

La réaction d'isomérisation du glucose en fructose est réalisée dans un autoclave de 100ml en mettant en oeuvre une solution à différents pourcentages en poids de glucose dans l'eau. Les quantités suivantes sont introduites dans le réacteur : 30g d'eau distillée, 0,3g de glucose (1 % en poids /eau) ou 0,6g de glucose (2% en poids/eau) ou 1,5g de glucose (5% en poids/eau) ou 3g de glucose (10% en poids/eau), 5% en poids de catalyseur/glucose. Aucune activation n'a été appliquée au catalyseur solide. 20 bars d'hélium sont introduits dans l'autoclave. Le milieu réactionnel est agité au moyen d'un agitateur magnétique. Le milieu réactionnel est porté à 100 °C au moyen de résistances électriques régulées à 100 °C. Après 2h de réaction à 100 °C, l'autoclave est refroidi au moyen d'un bain de glace. La conversion du glucose et le rendement molaire en fructose sont déterminés par analyse HPLC-RID (colonne : COREGEL 87C).

L'isomérisation a été réalisée en présence de 1%, 2%, 5% et 10% en poids de glucose par rapport au poids d'eau.

| | Conversion | Rdt Fructose (%) | Rdt Autres (%) |
|---|---|---|---|
| 1%Glucose | 52,6 | 28,2 | 11,6 |
| 2%Glucose | 35,6 | 25,6 | 7,4 |
| 5%Glucose | 47,3 | 28,6 | 11,5 |
| 10%Glucose | 47,9 | 29,1 | 13,2 |

| | | | |
|---|---|---|---|
| Rdt = rendement molaire | | | |

Les résultats montrent que la concentration en glucose dans le milieu réactionnel ne modifie pas de façon significative les résultats en termes de conversion du glucose, rendement et sélectivité en fructose.

### Exemple 7 : Isomérisation du glucose en fructose catalysé par MgLaO influence de la masse de catalyseur

La réaction d'isomérisation du glucose en fructose est réalisée dans un autoclave de 100ml en mettant en oeuvre une solution de 2% en poids de glucose dans l'eau. Les quantités suivantes sont introduites dans le réacteur : 30g d'eau distillée, 0,6g de glucose (2% en poids /eau), 0,03g de catalyseur (5% en poids / glucose) ou 0,6g de catalyseur (10% en poids /glucose) ou 0,12g de catalyseur (20% en poids/glucose) ou 0,3g de catalyseur (50% en poids/glucose) ou 0,6g de catalyseur (100% en poids /glucose). Aucune activation n'a été appliquée aux catalyseurs solides. 20 bars d'hélium sont introduits dans l'autoclave. Le milieu réactionnel est agité au moyen d'un agitateur magnétique. Le milieu réactionnel est porté à la température de réaction au moyen de résistances électriques régulées à 100 °C. Après 1h de réaction à 100 °C, l'autoclave est refroidi au moyen d'un bain de glace. La conversion du glucose et le rendement molaire en fructose sont déterminés par analyse HPLC-RID (colonne : COREGEL 87C).

L'isomérisation a été réalisée en présence de 5%, 10%, 20%, 50% et 100% en poids de MgLaO par rapport au poids de glucose.

| | Conversion (%) | Rdt Fructose (%) | Rdt Autres (%) |
|---|---|---|---|
| 5%MgLaO | 34,5 | 27,4 | 7,7 |
| 10%MgLaO | 38,7 | 31,2 | 8,6 |
| 20%MgLaO | 43,7 | 28,4 | 10,6 |
| 50%MgLaO | 56,2 | 29,0 | 18,9 |
| 100%MgLaO | 60,8 | 26,0 | 20,6 |

| | | | |
|---|---|---|---|
| Rdt = rendement molaire | | | |

Les résultats montrent qu'une augmentation de la masse de catalyseur par rapport au glucose permet d'augmenter la conversion du glucose. Cependant, le rendement et la sélectivité en fructose diminuent. Le meilleur compromis entre conversion du glucose et sélectivité en fructose sont obtenus pour des quantités de catalyseur inférieures à 20% en poids par rapport au poids de glucose.

### Exemple 8 : Résistance à l'empoisonnement de MgLaO par le CO₂

La réaction d'isomérisation du glucose en fructose est réalisée dans un autoclave de 100ml en mettant en oeuvre une solution de 2% en poids de glucose dans l'eau. Les quantités suivantes sont introduites dans le réacteur : 30g d'eau distillée, 0,6g de glucose (2% en poids /eau), 0,03g de catalyseur (5% en poids / glucose). Le catalyseur a été utilisé sans activation ou après les étapes d'activations suivantes : 0,03g de catalyseur sont traités sous vide secondaire à 400 °C pendant 2h, puis introduits rapidement dans le milieu réactionnel sans remise à l'air. Une autre étape d'activation du catalyseur a aussi consisté à mettre en contact le catalyseur avec 10 torrs de CO₂, suite au traitement sous vide secondaire à 400 °C pendant 2h. Le milieu réactionnel est agité au moyen d'un agitateur magnétique. Le milieu réactionnel est porté à la température de réaction au moyen de résistances électriques régulées à 100 °C. Après 1h de réaction à 100 °C, l'autoclave est refroidi au moyen d'un bain de glace. La conversion du glucose et le rendement molaire en fructose sont déterminés par analyse HPLC-RID (colonne : COREGEL 87C).

L'isomérisation a été réalisée avec du MgLaO non traité, du MgLaO traité à 400 °C sous vide pendant 2 heures et du MgLaO traité à 400 °C sous vide pendant 2 heures, puis sous 10 torrs de CO₂. Habituellement, le traitement à 400 °C sous vide permet d'activer un catalyseur.

| | Conversion (%) | Rdt Fructose (%) | Rdt Autres (%) |
|---|---|---|---|
| 5%MgLaO traite à 400 °C sv//2h | 31,7 | 23,5 | 6,5 |
| Mg LaO | 35,8 | 25,4 | 6,5 |
| 5%MgLaO traite à 400 °C sv//2h + CO2 | 33,1 | 24,9 | 6,3 |

| | | | |
|---|---|---|---|
| Rdt = rendement molaire, sv//2h = traitement sous vide pendant 2 heures | | | |

Les résultats ne montrent pas de différence significative en termes de conversion de glucose, rendement et sélectivité en fructose selon que le MgLaO ait été traité ou non et ait été mis au contact de CO₂. Ces résultats montrent donc que le MgLaO est résistant à l'empoisonnement par le CO₂.

### Exemple 9 : Cinétique de la réaction catalysée par MgLaO et MCM50

La réaction d'isomérisation du glucose en fructose est réalisée dans un autoclave de 100ml en mettant en oeuvre une solution de 1% ou 2% en poids de glucose dans l'eau. Les quantités suivantes sont introduites dans le réacteur : 30g d'eau distillée, 0,3g de glucose (1% en poids /eau) ou 0,6g de glucose (2% en poids/eau), 5% ou 20% en poids de catalyseur par rapport au glucose. Le catalyseur a été utilisé sans activation. Le milieu réactionnel est agité au moyen d'un agitateur magnétique. Le milieu réactionnel est porté à la température de réaction au moyen de résistances électriques régulées à 100 °C. Le milieu réactionnel est régulièrement échantillonné pour déterminer les évolutions de la conversion du glucose et du rendement en fructose au cours de la réaction. Après 2h de réaction à 100 °C, l'autoclave est refroidi au moyen d'un bain de glace. La conversion du glucose et le rendement molaire en fructose sont déterminés par analyse HPLC-RID (colonne: COREGEL 87C).

L'isomérisation a été réalisée avec du MgLaO à 5% en poids par rapport au poids de glucose et à 20% en poids par rapport au poids de glucose et avec du [CTA]Si-MCM50 à 5% en poids par rapport au poids du glucose, à 100 °C.

| | Conversion (%) | Rdt Fructose (%) | Rdt Mannose (%) | Rdt Autres (%) |
|---|---|---|---|---|
| 5%MgLaO | 8,2 (5 min) | 7,3 | 0,3 | 0 |
| 5%MgLaO | 20,2 (15 mn) | 18,2 | 1,4 | 1,1 |
| 5%MgLaO | 25,9 (30 min) | 21.0 | 2.0 | 1,5 |
| 5%MgLaO | 34,5 (60 min) | 27,4 | 3,5 | 4,1 |
| 5%MgLaO | 35,6(120 min) | 25,6 | 4,0 | 3,3 |
| 20%MgLaO | 43,7 (60 min) | 28,4 | 4,4 | 6,2 |
| 20%MgLaO | 55,8(120 min) | 26,9 | 4,8 | 7,1 |

| | | | | |
|---|---|---|---|---|
| Rdt = rendement molaire ; 2% en poids de Glucose/eau Rdt = rendement molaire ; 2% en poids de glucose/eau | | | | |

| | Conversion (%) | Rdt Fructose (%) | Rdt Mannose (%) | Rdt Autres (%) |
|---|---|---|---|---|
| 5%MCM50 | 9,8(30 min) | 8,8 | 0,5 | 0,16 |
| 5%MCM50 | 14,2(60min) | 12,1 | 0,6 | 0,5 |
| 5%MCM50 | 17,6(90min) | 15,4 | 0,9 | 0,9 |
| 5%MCM50 | 21,6(120 mini) | 16,0 | 1,1 | 1,1 |

| | | | | |
|---|---|---|---|---|
| Rdt = rendement molaire ; 1% en poids de glucose dans l'eau | | | | |

Les résultats montrent qu'une augmentation du temps de réaction permet une augmentation de la conversion au glucose qui se fait cependant au détriment de la sélectivité. Les résultats montrent qu'au bout d'une heure de réaction de bons rendements en fructose sont obtenus qui sont quasiment équivalents à ce qui peut être obtenu avec le procédé enzymatique.

### Exemple 10 : Recyclage du catalyseur

La réaction d'isomérisation du glucose en fructose est réalisée dans un autoclave de 100ml en mettant en oeuvre une solution de 2% en poids de glucose dans l'eau. Les quantités suivantes sont introduites dans le réacteur : 30g d'eau distillée, 0,6g de glucose (2% en poids /eau), 0,030g de catalyseur (5% en poids / glucose). Aucune activation n'a été appliquée aux catalyseurs solides. 20 bars d'hélium sont introduits dans l'autoclave. Le milieu réactionnel est agité au moyen d'un agitateur magnétique. Le milieu réactionnel est porté à la température de réaction au moyen de résistances électriques régulées à 100 °C. Après 1h de réaction à 100 °C, l'autoclave est refroidi au moyen d'un bain de glace, le catalyseur est séparé par filtration afin d'être réutilisé. le catalyseur est réintroduit dans l'autoclave sans traitement particulier, on introduit à nouveau dans l'autoclave les quantités suivantes : 30g d'eau distillée, 0,6g de glucose (2% en poids /eau). 3 cycles de tests de recyclage du catalyseur ont été réalisés de façon identique. La conversion du glucose et le rendement molaire en fructose sont déterminés par analyse HPLC-RID (colonne: COREGEL 87C).

| | Conversion (%) | Rdt Fructose (%) | Rdt Autres (%) |
|---|---|---|---|
| 1ere usage | 38,7 | 31,2 | 8,6 |
| 2eme usage | 27,6 | 22,4 | 4,7 |
| 3eme usage | 30,3 | 23,4 | 5,2 |
| 4eme usage | 29,8 | 23,5 | 5,2 |

| | | | |
|---|---|---|---|
| Rdt= rendement molaire | | | |

Les résultats montrent que le catalyseur peut être réutilisé pour plusieurs isomérisations sans traitement préalable. Aucune incidence sur la conversion du glucose, le rendement et la sélectivité en fructose n'est observée même après 4 utilisations du catalyseur sans traitement.

### Exemple 11 : Isomérisation en présence d'un solvant

La réaction d'isomérisation du glucose en fructose est réalisée dans un autoclave de 100ml en mettant en oeuvre une solution de 1% en poids de glucose dans l'eau. Les quantités suivantes sont introduites dans le réacteur : 15g d'eau distillée, 15g de DMSO, 0,3g de glucose (1% en poids /eau+DMSO), 0,015g de catalyseur (5% en poids / glucose) ou 0,03g de catalyseur (10% en poids/eau). Aucune activation n'a été appliquée aux catalyseurs solides. 20 bars d'hélium sont introduits dans l'autoclave. Le milieu réactionnel est agité au moyen d'un agitateur magnétique. Le milieu réactionnel est porté à la température de réaction au moyen de résistances électriques régulées à 100°C. Après 1h ou 2h ou 14h de réaction à 100 °C, l'autoclave est refroidi au moyen d'un bain de glace. La conversion du glucose et le rendement molaire en fructose sont déterminés par analyse HPLC-RID (colonne : COREGEL 87C).

La réaction d'isomérisation a été effectuée avec différentes quantité de catalyseur dans un milieu réactionnel eau + DMSO :
- 5% MgLaO en poids par rapport au poids de glucose avec 50% DMSO en poids par rapport au poids total de milieu eau+DMSO ;
- 10% MgLaO en poids par rapport au poids de glucose avec 50% DMSO en poids par rapport au poids total de milieu eau+DMSO ;
- 5% MgLaO en poids par rapport au poids de glucose avec 50% DMSO en poids par rapport au poids total de milieu eau+DMSO pendant 14 heures.

| | Conversion (%) | Rdt Fructose (%) | Rdt Mannose (%) | Rdt Autres (%) |
|---|---|---|---|---|
| 5%MgLaO, 50%DMSO Réaction 1 heure | 13,2 | 12,1 | 0,7 | 0 |
| 10%MgLaO, 50%DMSO Réaction 2 heures | 23,9 | 19,1 | 2,5 | 0,7 |
| 5%MgLaO, 50%DMSO Réaction 14 heures | 31,6 | 26,1 | 3,7 | 0 |

| | | | | |
|---|---|---|---|---|
| Rdt = rendement molaire, | | | | |

Les résultats montrent que la réaction peut être conduite en solvant non totalement aqueux, à savoir en présence de solvant organique.

### Exemple 12 : Variation du catalyseur

La réaction d'isomérisation du glucose en fructose est réalisée dans un autoclave de 100ml en mettant en oeuvre une solution de 2% en poids de glucose dans l'eau. Les quantités suivantes sont introduites dans le réacteur : 30g d'eau distillée, 0,6g de glucose (2% en poids /eau), 5% ou 10%en poids de catalyseur par rapport au poids de glucose. Aucune activation n'a été appliquée aux catalyseurs solides. 20 bars d'hélium sont introduits dans l'autoclave. Le milieu réactionnel est agité au moyen d'un agitateur magnétique. Le milieu réactionnel est porté à la température de réaction au moyen de résistances électriques régulées à 100 °C. Après 1h de réaction à 100 °C, l'autoclave est refroidi au moyen d'un bain de glace. La conversion du glucose et le rendement molaire en fructose sont déterminés par analyse HPLC-RID (colonne : COREGEL 87C).

L'isomérisation a été réalisée en présence de différents catalyseurs, à savoir :
- MgLaO (oxyde mixte à base de magnésium et de lanthane)
- CeMgO (oxyde mixte à base de magnésium et de cerium)
- HY (zéolite ne comprenant plus de template organique) : exemple comparatif
- ZSM5 (zéolite ne comprenant plus de template organique) : exemple comparatif

| Catalyseur | Conversion | Rdt Fructose | Rdt Autres |
|---|---|---|---|
| LaMgO * | 38,67 | 31,24 | 8,6 |
| LaMgO | 34,54 | 27,36 | 7,68 |
| CeMgO | 27,44 | 20,35 | 3,64 |
| HY | 7,32 | 2,32 | 0 |
| ZSM5 | 3,05 | 1,06 | 0,48 |

| | | | |
|---|---|---|---|
| Rdt = rendement molaire, 2%Glucose, 1 h, 100 °C, 5%catalyseur, *10% catalyseur | | | |

Ces résultats montrent qu'il est possible de mettre en oeuvre le procédé de l'invention avec divers lanthanides tout en obtenant de bons résultats en termes de conversion et de rendement en fructose.

### Exemple 13 : Préparation HMF à partir de glucose

La réaction de conversion du glucose en HMF est réalisée en intégrant deux étapes dans un seul autoclave: une première étape d'isomérisation du glucose en fructose en présence de MgLaO à 100 °C (ou à 150 °C) en milieu 100% eau pendant 2h, suivi par une deuxième étape de déshydratation du fructose en HMF en présence d'acides carboxyliques. La synthèse de l'HMF est réalisée dans un autoclave de 100ml en mettant en oeuvre une solution de 1% en poids de glucose dans l'eau. Les quantités suivantes sont introduites dans le réacteur : 15g d'eau distillée, 0,3g de glucose (2% en poids /eau), 0,015g de catalyseur (5% en poids / glucose). Aucune activation n'a été appliquée au catalyseur MgLaO. 20 bars d'hélium sont introduits dans l'autoclave. Le milieu réactionnel est agité au moyen d'un agitateur magnétique. Le milieu réactionnel est porté à la température de réaction au moyen de résistances électriques régulées à 100°C pendant 2h (ou à 150 °C pendant 1,5h). Puis la deuxième étape débute par l'ajout au milieu réactionnel de 15g d'acide acétique. La réaction est poursuivie pendant 2h à 150 °C. En fin de réaction, l'autoclave est refroidi au moyen d'un bain de glace. La conversion du glucose et le rendement molaire en fructose sont déterminés par analyse HPLC-RID (colonne: COREGEL 87C).

| | Conversion (%) | Rdt HMF (%) | Rdt Fructose (%) | Rdt Mannose (%) | Rdt Autres (%) |
|---|---|---|---|---|---|
| **étape 1 :** MgLaO-100% eau-150 °C-1h30 | 56,5 | 16,8 | 3,1 | 6,2 | 2,4 |
| **étape 2 :** 50% acide acétique/50% eau-150 °C-2h | | | | | |
| **étape 1 :** MgLaO-100% eau-100 °C-2h | 33,5 | 15,4 | 1,1 | 1,3 | 2,1 |
| **étape 2 :** 50% acide acétique/50% eau-150 °C-2h | | | | | |

| | | | | | |
|---|---|---|---|---|---|
| Rdt = rendement molaire | | | | | |

Les résultats montrent que le procédé selon l'invention permet de préparer le HMF avec un rendement convenable à partir du glucose.

### Exemple 14 : Etude de la réversibilité de l'adsorption du CO₂

La réversibilité de l'adsorption de CO₂ par un catalyseur peut être déterminée par mesures d'isothermes d'adsorption du CO₂ (totale et réversible) par mesure volumétrique couplée à la calorimétrie. Les mesures d'adsorption du CO₂ sont réalisées au moyen d'un calorimètre Tian Calvet, elles sont couplées aux mesures d'isothermes d'adsorption du CO₂ au moyen d'un équipement volumétrique en pyrex équipé d'une jauge de pression de précision. Le solide est introduit dans la cellule du calorimètre maintenu à la température de mesure (30 °C) pour être prétraité sous vide à cette température jusqu'à l'obtention d'un bon vide (10⁻⁵ torrs). Le CO₂ est introduit par petits incréments à 30 °C, la chaleur dégagée est mesurée à chaque dose de CO₂ tout comme la pression d'équilibre. L'isotherme d'adsorption est déterminée jusqu'à la pression d'équilibre de 1 Torr. Après la mesure de la première isotherme (adsorption totale) réalisée parallèlement à la première courbe calorimétrique, une seconde isotherme est réalisée (adsorption réversible) et en parallèle la seconde courbe calorimétrique après avoir traité sous vide le catalyseur solide à 30 °C pendant 15h. On conclut que l'adsorption de CO₂ est réversible lorsque l'on observe que les isothermes (totale et réversible) sont superposables et que les courbes calorimétriques (totale et reversible) sont superposable en acceptant un écart inférieur à 20%.

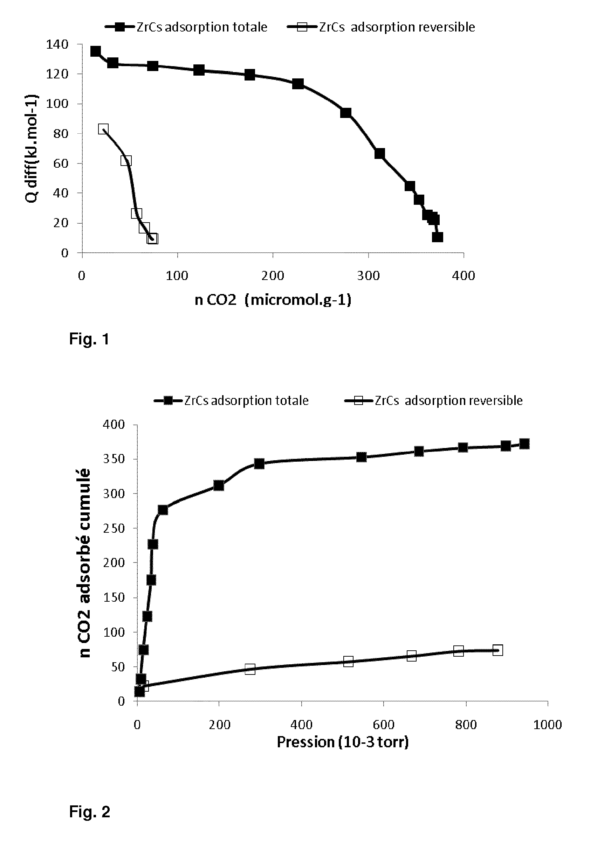

Les résultats obtenus pour les catalyseurs ZrCs (comparatif), MCM-48, MCM-50 sont présentés aux figures 1 à 4.

La figure 1 représente les courbes calorimétriques d'adsorption du CO₂ pour un catalyseur ZrCs activé sous vide à 450 °C pendant 2 heures. Les carrés noirs montrent l'évolution de la chaleur différentielle d'adsorption du CO₂ avec le recouvrement en CO₂ lors de la première adsorption (adsorption totale), les carrés blancs montrent l'évolution des chaleurs différentielles d'adsorption du CO₂ avec le recouvrement en CO₂ lors de la deuxième adsorption (adsorption réversible), c'est-à-dire réalisée après un traitement sous vide du catalyseur à 30 °C pendant 15h suite à la première adsorption de CO₂. On note que la zircone dopée au Cs, ZrCs, activée sous vide à 450 °C, présente des sites basiques forts, caractérisés par une distribution homogène en force et par une chaleur différentielle d'adsorption du CO₂ de l'ordre de 125-130 kJ.mol-1. La deuxième courbe calorimétrique obtenue après une première adsorption de CO₂ suivie par un traitement sous vide à 30 °C pendant 15h montre la non réversibilité de l'adsorption de CO₂ sur ZrCs activée à 450 °C sous vide. La deuxième courbe calorimétrique est caractérisée par une chaleur initiale différentielle d'adsorption de CO₂ de 80 kJ.mol⁻¹ qui décroît rapidement avec le recouvrement en CO₂. Les deux courbes calorimétriques ne sont pas superposables, ceci montre la non réversibilité de l'adsorption de CO₂ de ZrCs activé à 450 °C.

La figure 2 montre les isothermes d'adsorption du CO₂ mesurées parallèlement à la mesure des chaleurs différentielles d'adsorption du CO₂ avec le recouvrement en CO₂ lors de la première et de la deuxième adsorption du CO₂ (respectivement adsorptions totale et réversible). Ces courbes montrent la non réversibilité de l'adsorption de CO₂ sur ZrCs activée sous vide à 450 °C : alors que l'adsorption totale correspond à une adsorption de 350 micromol.g⁻¹ de CO₂, l'adsorption réversible est limitée à 50 micromole.g⁻¹ de CO₂. Ceci montre que les deux isothermes ne se superposent pas sur ZrCs activée à 450 °C sous vide, l'adsorption de CO₂ sur ZrCs activée à 450 °C est non réversible.

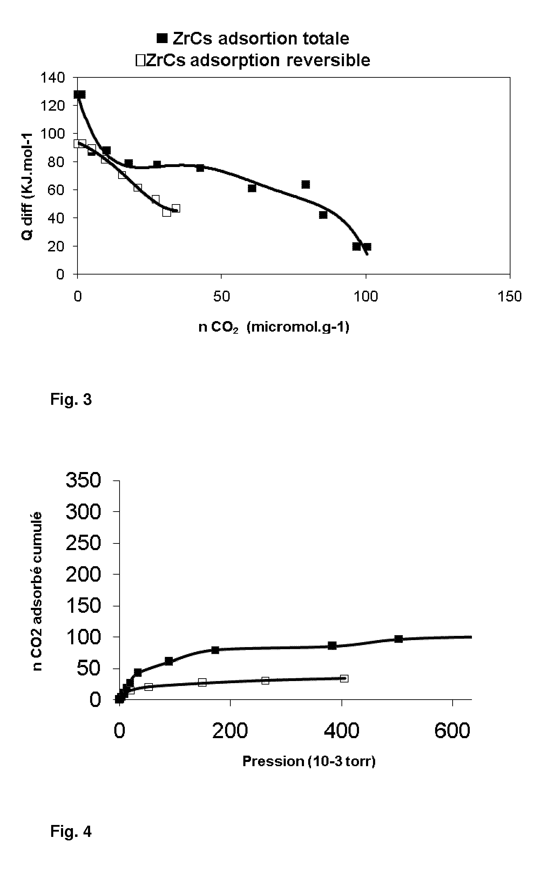

La figure 3 représente les courbes calorimétriques d'adsorption du CO₂ pour un catalyseur ZrCs prétraité sous vide à 30 °C pendant 24 heures. Les carrés noirs montrent l'évolution de la chaleur différentielle d'adsorption du CO₂ avec le recouvrement en CO₂ lors de la première adsorption (adsorption totale), les carrés blancs montrent l'évolution des chaleurs différentielles d'adsorption du CO₂ avec le recouvrement en CO₂ lors de la deuxième adsorption (adsorption réversible) c'est-à-dire réalisée après un traitement sous vide du catalyseur à 30°C pendant 15h suite à la première adsorption de CO₂ On note que la zircone dopée au Cs, ZrCs, activée sous vide à 30 °C, présente quelques sites basiques forts, caractérisés par une chaleur différentielle d'adsorption du CO₂ de l'ordre de 125-130 kJ.mol⁻¹ et une majorité de sites basiques plus faibles montrant une chaleur différentielle d'adsorption du CO₂ de l'ordre de 80 kJ.mol⁻¹. Une comparaison avec la deuxième courbe calorimétrique obtenue après une première adsorption de CO₂ suivie par un traitement sous vide à 30 °C pendant 15h montre la non réversibilité de l'adsorption de CO₂ sur ZrCs activée à 30°C sous vide. La deuxième courbe calorimétrique est caractérisée par une chaleur initiale différentielle d'adsorption de CO₂ de 90 kJ.mol⁻¹ qui décroît rapidement avec le recouvrement en CO₂. Les deux courbes calorimétriques ne sont pas superposables, ceci montre la non réversibilité de l'adsorption de CO₂ de ZrCs activée sous vide à 30 °C pendant 24h.

La figure 4 montre les isothermes d'adsorption du CO₂ mesurées parallèlement à la mesure des chaleurs différentielles d'adsorption du CO₂ avec le recouvrement en CO₂ lors de la première et de la deuxième adsorption du CO₂ (respectivement adsorptions totale et réversible). Ces courbes montrent la non réversibilité de l'adsorption de CO₂ sur ZrCs prétraité sous vide à 30 °C : alors que l'adsorption totale correspond à une adsorption de 100 micromol.g⁻¹ de CO₂, l'adsorption réversible est limitée à 25 micromole.g⁻¹ de CO₂ montrant que les deux isothermes ne se superposent pas sur ZrCs prétraité à 30 °C sous vide pendant 24h.

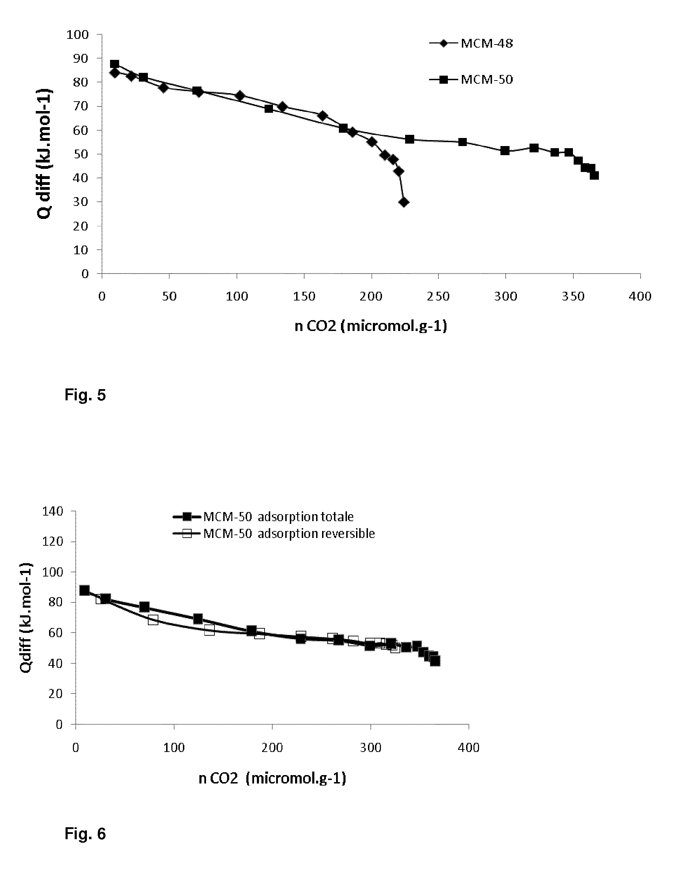

La figure 5 représente les courbes calorimétriques d'adsorption du CO₂ (première adsorption totale) pour des catalyseurs [CTA]Si-MCM48, et [CTA]Si-MCM50.

La figure 6 représente les courbes calorimétriques d'adsorption du CO₂ pour un catalyseur [CTA]Si-MCM50 prétraité sous vide à 30 °C pendant 24 heures. Les carrés noirs montrent l'évolution de la chaleur différentielle d'adsorption du CO₂ avec le recouvrement en CO₂ lors de la première adsorption (adsorption totale), les carrés blancs montrent l'évolution des chaleurs différentielles d'adsorption du CO₂ avec le recouvrement en CO₂ lors de la deuxième adsorption (adsorption réversible), c'est-à-dire réalisée après un traitement sous vide du catalyseur à 30 °C pendant 15h suite à la première adsorption de CO₂. On note que le catalyseur [CTA]Si-MCM50, activé sous vide à 30 °C pendant 24h, présente des sites basiques faibles, caractérisés par une chaleur différentielle d'adsorption du CO₂ de l'ordre de 75-80 kJ.mol⁻¹. La deuxième courbe calorimétrique (adsorption réversible) obtenue après une première adsorption de CO₂ suivie par un traitement sous vide à 30 °C pendant 15h est quasiment similaireà la première courbe calorimétrique (adsorption totale). Les deux courbes calorimétriques sont superposables,

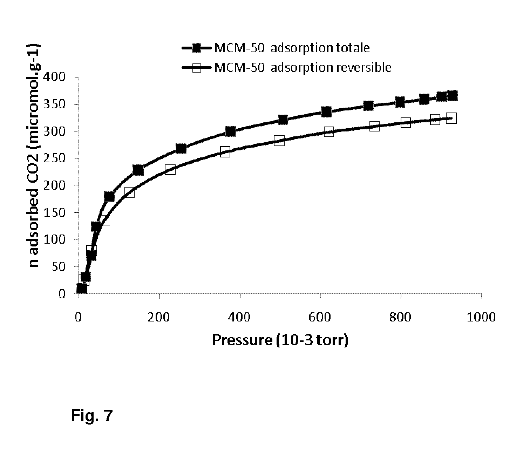

La figure 7 montre les isothermes d'adsorption du CO₂ mesurées sur [CTA]Si-MCM50 prétraité sous vide à 30 °C parallèlement à la mesure des chaleurs différentielles d'adsorption du CO₂ avec le recouvrement en CO₂ lors de la première et de la deuxième adsorption du CO₂ (respectivement adsorptions totale et réversible). Alors que l'adsorption totale correspond à une adsorption de 360 micromol.g⁻¹ de CO₂, l'adsorption réversible atteint une valeur très proche de 325 micromole.g⁻¹ de CO₂ soit un écart inférieur à 10%. Ceci montre que les deux isothermes sont quasiment superposables sur [CTA]Si-MCM50 prétraité sous vide à 30 °C.
Puisque les courbes calorimétriques d'adsorption du CO₂ (totale et réversible) sont superposables et que les isothermes d'adsorption du CO₂ (totale et réversible) sont superposables sur [CTA]Si-MCM50 prétraité sous vide à 30 °C, on conclut que l' adsorption de CO₂ sur [CTA]Si-MCM50 est réversible.

## Revendications

1. Procédé d'isomérisation du glucose en fructose dans l'eau en présence d'un catalyseur basique solide, **caractérisé par** la réversibilité des isothermes d'adsorption du CO₂ à basse température, notamment à 30°C et une chaleur différentielle d'adsorption du CO₂, mesurée à 30°C, comprise entre 60 et 110 kJ.mol⁻¹, de façon préférée entre 75 et 90kJ.mol⁻¹, le catalyseur étant un catalyseur comprenant au moins un oxyde de scandium ou un oxyde de Lanthanide choisi parmi les éléments chimiques de numéro atomique 57 à 71 supporté ou non supporté ou un tamis moléculaire à base de silicium contenant son template organique.

2. Procédé selon la revendication 1, pour lequel le catalyseur est choisi parmi les oxydes de Lanthanides, supportés ou non, éventuellement hydroxylés et/ou carbonatés, les oxydes mixtes de Lanthanides avec d'autres métaux, supportés ou non, éventuellement hydroxylés et/ou carbonatés, ou les tamis moléculaires à base de silicium contenant leur template organique.

3. Procédé selon la revendication 1 ou 2, pour lequel le catalyseur est choisi parmi les oxydes mixtes de Lanthanides avec d'autres métaux, alcalins ou alcalino-terreux ou les oxydes de Lanthanides supportés, les supports étant des supports carbonés ou des oxydes métalliques.

4. Procédé selon l'une des revendications 1 à 3, pour lequel le catalyseur est MgLaO ou LaO supporté sur charbon.

5. Procédé selon la revendication 1, pour lequel le catalyseur est un tamis moléculaire de la famille M41 S, de préférence MCM48 ou MCM50, contenant leur template organique.

6. Procédé selon l'une des revendications 1 à 5, pour lequel la quantité de catalyseur est comprise entre 0,5 et 100% en poids, de préférence entre 1 et 50% en poids, notamment entre 2 et 25% en poids, par exemple entre 5 et 10% en poids, par rapport au poids de glucose.

7. Procédé selon l'une des revendications 1 à 6, pour lequel la quantité de glucose est comprise entre 0,5 et 15% en poids, de préférence entre 1 et 10% en poids, par rapport au poids d'eau.

8. Procédé selon l'une quelconque des revendications 1 à 7, le procédé étant mis en oeuvre à une température comprise entre 75 et 180°C, de préférence entre 80 et 150°C, par exemple à 100°C.

9. Procédé selon l'une quelconque des revendications 1 à 8, ne comprenant pas de prétraitement, notamment prétraitement thermique du catalyseur.

10. Procédé selon l'une des revendications 1 à 8, comprenant une étape préalable de traitement du catalyseur à la pyridine.

11. Procédé de préparation du 5-hydroxyméthylfurfural (HMF) à partir de glucose comprenant les étapes suivantes :
i) isomérisation du glucose en fructose selon le procédé selon l'une quelconque des revendications 1 à 10 ;
ii) ajout d'acide carboxylique au milieu réactionnel obtenu à l'étape i) ;
iii) récupération du HMF.

12. Procédé selon la revendication 11, pour lequel les acides sont :
- des acides de formule R-COOH dans laquelle R représente un atome d'hydrogène ou une chaîne alkyle, linéaire ou ramifiée, en C₁ à C₅, de préférence en C₁ à C₃, éventuellement substituée par un ou plusieurs groupes OH ;
- des acides de formule HOOC-L-COOH dans laquelle L représente une liaison ou une chaîne alkyle, linéaire ou ramifiée, en C₁ à C₅, de préférence en C₁ à C₃, éventuellement substituée par un ou plusieurs groupes OH et/ou COOH ; ou
- leurs mélanges.

13. Procédé selon l'une des revendications 11 ou 12, pour lequel l'acide carboxylique est l'acide formique, l'acide acétique, l'acide malique, l'acide citrique, l'acide oxalique, l'acide lactique ou leurs mélanges, de préférence il s'agit de l'acide acétique.

14. Procédé selon l'une des revendications 11 à 13, pour lequel la quantité d'acide est inférieure à 80 % en poids, généralement comprise entre 5 et 80 % en poids, notamment comprise entre 10 et 60 % en poids, par exemple 50%, 20 % ou 10 % en poids par rapport au poids de la solution aqueuse d'acides carboxyliques.

15. Procédé selon l'une des revendications 11 à 14, l'étape (ii) étant menée à une température comprise entre 100 et 200 °C, de préférence entre 120 et 180 °C, par exemple entre 150 et 180 °C.

16. Procédé selon l'une des revendications 11 à 15, l'étape (ii) étant mise en oeuvre en présence d'un catalyseur acide hétérogène, notamment choisi parmi l'acide 12-tungstophosphorique, de préférence dispersé sur hydroxyde de niobium (NbOH); l'hydroxyde de niobium ; les zeolithes telles H-ZSM-5 ou H-Y, des argiles cationiques de type K10; les charbons sulfonés ; les charbons fonctionnalisés, par exemple par des groupements carboxyliques, par exemple suite à une oxydation, par exemple oxydation par de l'eau de javel ; ou leurs mélanges.

17. Procédé selon la revendication 16, pour lequel la quantité de catalyseur est de préférence comprise entre 2 et 100% en poids, de préférence entre 2 et 10 % en poids, par exemple 5 % en poids par rapport au poids de glucose de départ.

## Patentansprüche

1. Verfahren zur Isomerisierung von Glukose zu Fruktose in Wasser in Gegenwart eines festen basischen Katalysators,
**gekennzeichnet durch** die Reversibilität der Adsorptionsisothermen von CO₂ bei niedriger Temperatur, insbesondere bei 30 °C, und eine differentielle Adsorptionswärme von CO₂ von 60 bis 110 kJ.mol⁻¹, vorzugsweise 75 bis 90 kJ.mol⁻¹, wobei der Katalysator ein Katalysator ist, der zumindest Folgendes aufweist: ein Scandiumoxid oder ein Lanthanidoxid, das aus den chemischen Elementen der Atomzahl 57 bis 71 ausgewählt ist, das trägergebunden oder nicht trägergebunden ist, oder ein Molekularsieb auf der Basis von Silicium, das sein organisches Templat enthält.

2. Verfahren nach Anspruch 1,
wobei der Katalysator ausgewählt ist aus Oxiden von Lanthaniden, die trägergebunden sind oder nicht, gegebenenfalls hydroxyliert und/oder carbonatiert sind, aus Mischoxiden von Lanthaniden mit anderen Metallen, die trägergebunden sind oder nicht, gegebenenfalls hydroxyliert und/oder carbonatiert sind, oder aus Molekularsieben auf der Basis von Silicium, die ihr organisches Templat enthalten.

3. Verfahren nach Anspruch 1 oder 2,
wobei der Katalysator ausgewählt ist aus Mischoxiden von Lanthaniden mit anderen Metallen, Alkali- oder Erdalkalimetallen oder trägergebundenen Oxiden von Lanthaniden, wobei die Träger carbonierte Träger oder Metalloxide sind.

4. Verfahren nach einem der Ansprüche 1 bis 3,
wobei der Katalysator MgLaO oder LaO trägergebunden auf Kohle ist.

5. Verfahren nach Anspruch 1,
wobei der Katalysator ein Molekularsieb der Familie M41 S, vorzugsweise MCM48 oder MCM50 ist, die ihr organisches Templat enthalten.

6. Verfahren nach einem der Ansprüche 1 bis 5,
wobei die Menge an Katalysator 0,5 bis 100 Gew.-%, vorzugsweise 1 bis 50 Gew.-%, insbesondere 2 bis 25 Gew.-%, beispielsweise 5 bis 10 Gew.-%, bezogen auf das Gewicht von Glukose, beträgt.

7. Verfahren nach einem der Ansprüche 1 bis 6,
wobei die Menge an Glucose 0,5 bis 15 Gew.-%, vorzugsweise 1 bis 10 Gew.-%, bezogen auf das Gewicht von Wasser beträgt.

8. Verfahren nach einem der Ansprüche 1 bis 7,
wobei das Verfahren bei einer Temperatur von 75 °C bis 180 °C, vorzugsweise von 80 °C bis 150 °C, beispielsweise bei 100 °C durchgeführt wird.

9. Verfahren nach einem der Ansprüche 1 bis 8,
das keine Vorbehandlung, insbesondere keine Wärmevorbehandlung des Katalysators umfasst.

10. Verfahren nach einem der Ansprüche 1 bis 8,
das einen vorherigen Schritt der Behandlung des Katalysators mit Pyridin umfasst.

11. Verfahren zur Herstellung von 5-Hydroxymethylfurfural (HMF) aus Glucose, das die folgenden Schritte umfasst:
i) Isomerisierung der Glukose zu Fruktose mit dem Verfahren nach einem der Ansprüche 1 bis10,
ii) Zugabe von Carboxylsäure zu dem in Schritt i) erhaltenen Reaktionsmedium;
iii) Gewinnung des HMF.

12. Verfahren nach Anspruch 11, wobei die Säuren folgende sind:
- Säuren der Formel R-COOH, wobei R ein Wasserstoffatom oder eine lineare oder verzweigte C₁-C₅-, vorzugsweise eine C₁-C₃-Alkylkette darstellt, die gegebenenfalls mit einer oder mehreren OH-Gruppen substituiert ist;
- Säuren der Formel HOOC-L-COOH, wobei L eine Bindung oder eine lineare oder verzweigte C₁-C₅-, vorzugsweise eine C₁-C₃-Alkylkette darstellt, die gegebenenfalls mit einer oder mehreren OH-Gruppen und/oder COOH-Gruppen substituiert ist; oder
- Gemische von diesen.

13. Verfahren nach einem der Ansprüche 11 oder 12,
wobei die Carboxylsäure Ameisensäure, Essigsäure, Äpfelsäure, Citronensäure, Oxalsäure, Milchsäure oder ein Gemisch von diesen ist, vorzugsweise Essigsäure ist.

14. Verfahren nach einem der Ansprüche 11 bis 13,
wobei die Menge an Säure kleiner als 80 Gew.-% ist, im Allgemeinen 5 bis 80 Gew.-%, insbesondere 10 bis 60 Gew.-%, beispielsweise 50 Gew.-%, 20 Gew.-% oder 10 Gew.-%, bezogen auf das Gewicht der wässrigen Lösung von Carboxylsäuren, beträgt.

15. Verfahren nach einem der Ansprüche 11 bis 14,
wobei der Schritt (ii) bei einer Temperatur von 100 °C bis 200 °C, vorzugsweise von 120 °C bis 180 °C, beispielsweise bei 150 °C bis 180 °C durchgeführt wird.

16. Verfahren nach einem der Ansprüche 11 bis 15,
wobei der Schritt (ii) in Gegenwart eines heterogenen Säure-Katalysators durchgeführt wird, der insbesondere ausgewählt wird aus 12-Wolframphosphorsäure, vorzugsweise dispergiert auf Niobhydroxid (NbOH); Niobhydroxid; Zeolithen, wie H-ZSM-5 oder H-Y; kationischen Tonen vom Typ K10; sulfonierten Kohlen; funktionalisierten Kohlen, beispielsweise durch Carboxylgruppen, beispielsweise mit einer Oxidation, beispielsweise Oxidation durch Javelwasser; oder Gemischen von diesen.

17. Verfahren nach Anspruch 16,
wobei die Menge an Katalysator, bezogen auf das Gewicht Ausgangsglukose, vorzugsweise 2 bis 100 Gew.-%, vorzugsweise 2 bis 10 Gew.-%, beispielsweise 5 Gew.-% beträgt.

## Claims

1. A method for isomerisation of glucose into fructose in water in the presence of a solid basic catalyst, **characterized by** the reversibility of the CO₂ adsorption isotherms at low temperature, notably at 30°C, and a differential CO₂ adsorption heat, measured at 30°C, comprised between 60 and 110 kJ.mol⁻¹, preferably between 75 and 90 kJ.mol⁻¹, the catalyst being a catalyst comprising at least one scandium oxide or one lanthanide oxide selected from the chemical elements with atomic number from 57 to 71, either supported or not supported or a molecular sieve based on silicon containing its organic template.

2. The method according to claim 1, for which the catalyst is selected from lanthanide oxides, either supported or not, optionally hydroxylated and/or carbonated, mixed oxides of lanthanides with other metals, either supported or not, optionally hydroxylated and/or carbonated, or molecular sieves based on silicon containing their organic template.

3. The method according to claim 1 or 2, for which the catalyst is selected from mixed oxides of lanthanides with other alkaline or earth alkaline metals or supported oxides of lanthanides, the supports being carbonaceous supports or metal oxides.

4. The method according to one of claims 1 to 3, for which the catalyst is MgLaO or LaO supported on coal.

5. The method according to claim 1, for which the catalyst is a molecular sieve of the M41 S, preferably MCM48 or MCM50 family, containing their organic template.

6. The method according to one of claims 1 to 5, for which the catalyst amount is comprised between 0.5 and 100% by weight, preferably between 1 and 50% by weight, notably between 2 and 25% by weight, for example between 5 and 10% by weight, based on the weight of glucose.

7. The method according to one of claims 1 to 6, for which the glucose amount is comprised between 0.5 and 15% by weight, preferably between 1 and 10% by weight, based on the weight of water.

8. The method according to any of claims 1 to 7, the method being applied at a temperature comprised between 75 and 180°C, preferably between 80 and 150°C, for example at 100°C.

9. The method according to any of claims 1 to 8, not comprising any pretreatment, notably no thermal pretreatment of the catalyst.

10. The method according to one of claims 1 to 8, comprising a preliminary step for treating the catalyst with pyridine.

11. A method for preparing 5-hydroxymethylfurfural (HMF) from glucose comprising the following steps:
i) isomerisation of the glucose into fructose in accordance with the method according to any of claims 1 to 10;
ii) adding carboxylic acid to the reaction medium obtained in step i);
iii) recovery of HMF.

12. The method according to claim 11, for which the acids are:
- acids of formula R-COOH wherein R represents a hydrogen atom or a linear or branched C₁-C₅, preferably C₁-C₃, alkyl chain, optionally substituted with one or several OH groups;
- acids of formula HOOC-L-COOH wherein L represents a bond or a linear or branched C₁-C₅, preferably C₁-C₃, alkyl chain optionally substituted with one or several OH and/or COOH groups; or
- mixtures thereof.

13. The method according to one of claims 11 or 12, for which the carboxylic acid is formic acid, acetic acid, malic acid, citric acid, oxalic acid, lactic acid or mixtures thereof, preferably this is acetic acid.

14. The method according to one of claims 11 to 13, for which the amount of acid is less than 80% by weight, generally comprised between 5 and 80% by weight, notably comprised between 10 and 60% by weight, for example 50%, 20% or 10% by weight based on the weight of the aqueous solution of carboxylic acids.

15. The method according to one of claims 11 to 14, the step (ii) being conducted at a temperature comprised between 100 and 200°C, preferably between 120 and 180°C, for example between 150 and 180°C.

16. The method according to one of claims 11 to 15, the step (ii) being applied in the presence of a heterogeneous acid catalyst, notably selected from 12-tungstophosphoric acid, preferably dispersed on niobium hydroxide (NbOH); niobium hydroxide; zeolites such as H-ZSM-5 or H-Y, cationic clays of the K10 type; sulfonated coals; functionalized coals, for example with carboxylic groups, for example subsequently to oxidation, for example oxidation by sodium hypochlorite; or mixtures thereof.

17. The method according to claim 16, for which the amount of catalyst is preferably comprised between 2 and 100% by weight, preferably between 2 and 10% by weight, for example 5% by weight based on the initial weight of glucose.
